# EUROPEAN PATENT APPLICATION

(11) **EP 1 304 346 A2**
(43) Date of publication of application: **23.04.2003**
(21) Application number: 02079756.9
(22) Date of filing: 03.07.1998
(51) Int. Cl.: C08G 81/00, C08B 37/08, A61K 31/722, A61K 9/127, A61K 9/50, A61K 9/16

(54) **Conjugate of polyethylene glycol and chitosan**

(30) Priority: 03.07.1997 GB 9713980
(62) Divisional of application: 98932368.8
(71) Applicant: West Pharmaceutical Services Drug Delivery & Clinical Research Centre Limited, Nottingham NG7 2TN (GB)
(72) Inventor: Davis, Stanley Stewart, Nottingham NG7 1BA (GB); Lin, Wu, Lenton, Nottingham NG7 2EQ (GB); Bignotti, Fabio, 25081 Bedizzole (IT); Ferruti, Paolo, 2015 Milano (IT)
(74) Representative: McNeeney, Stephen Phillip

(57) **Abstract**

This invention relates to a composition for the systemic uptake of a drug across a mucosal surface. The composition comprises a PEG-chitosan conjugate.

## Description

The present invention concerns a novel conjugate between chitosan and polyethylene glycol (PEG-chitosan conjugate) that may be used in biomedical applications and particularly in the fields of antisense and gene therapy, drug absorption enhancement and targeting using particulate carriers.

Chitosan is a biopolymer material that is derived from chitin. Chemically, chitosan is a polyglucosamine, a linear polymer of β (1→4) linked 2-amino-2-deoxy-D glucopyranose. Chitosan can be derived from chitin through a process of deacetylation. While chitin is insoluble, chitosan in its salt form can demonstrate acceptable solubilities at pH's below 7.0. Chitosan is believed to be non-toxic and can be administered to mucosal surfaces.

Various applications for chitosan have been described in the prior art including its use in wound healing, controlled drug delivery, as a bioadhesive material and to improve the solubility of poorly soluble compounds. More recently it has been discovered that chitosan, because of its positive charge, can have an effect upon the tight junctions between cells and it has been shown to be effective in improving the paracellular transport of drugs, particularly those that are polar in character. A detailed review of the medical applications of chitosan can be found in the article by Hon in Polysaccharides in Medical Applications, Ed. Dumitru, S., Dekker, New York, 1996, pages 631-649. Specific examples of medical applications for chitosan can also be found in PCT/GB90/00291, in Artursson et al., Pharm. Res. 11 1358, 1994 and in Ilium et al. Pharm. Res. 11 1186, 1994.

Chitosan can be obtained in a range of molecular weights from oligomeric materials containing a few units of glucosamine through to higher molecular weight materials of more than 200,000 Daltons. In pharmaceutical applications, the higher molecular weights from 50,000 to 500,000 Daltons are normally preferred. Chitosan can also be obtained in different degrees of deacetylation, but the materials that have a deacetylation of between 60 and 90% are normally preferred. Chitosan can be obtained from various sources, including shell fish, fungi and other materials and a pharmaceutical grade of chitosan is available from Pronova Limited of Norway.

Because chitosan carries a positive charge, it can be used to interact with negatively charged surfaces as well as with other negatively charged materials including pharmaceuticals. Chitosan can also be used to modify the surfaces of carrier particles. For example, liposomes have been described where chitosan has been bound to their surfaces (Takeuchiu et al. (1994), Chem. Pharm. Bull. 42 1954-1956).

Chitosan can be employed in solution, as a powder material, in microspheres or can be used as a film-forming agent.

It is also known to modify chitosan chemically to produce derivatives with new properties. Examples include N-acylchitosans, N-carboxyalkyl chitosans, N-carboxyacyl chitosans and O-carboxyalkyl chitosans.

Simple admixtures of chitosan and polyethylene glycol (PEG) are known. For example, chitosan/poly(ethylene oxide) polymer networks have been described by Patel and Amiji (Polymer Preprints, ACS Division of Polymer Chem. 1994, 352, 403 and ACS Symposium Series, 627, Hydrogels and biodegradable polymers for bioapplications, page 209). The hydrogel was prepared by dissolving chitosan in acetic acid to produce a solution. Polyethylene glycol having a molecular weight of 10 kilodaltons (kD) to 1 megadalton (mD) was dissolved in acetic acid and the resulting solution added to the chitosan solution to prepare a physical blend. The blend was then cross-linked with glyoxal to form the gel system. There was no suggestion in the work of Patel and Amiji to bond polyethylene glycol, also known as polyethylene oxide, directly to chitosan to form a PEG-chitosan conjugate.

Chitosan/polyether hydrogels have also been described by Peng et al. (J. Polymer Science Part A, Polymer Chemistry 32, 591-596, 1994). Here also the chitosan and a polyether (polyoxypropylene glycol) were mixed at acidic pH and then cross-linked with glutaraldehyde.

The attachment of polyethylene glycol to polymeric materials including proteins, carbohydrates and phospholipids is known - see, for example, the article by Zalipsky, Advances in Drug Delivery Research, 16, 157-182 (1995). This process is often referred to as PEGylation and this term will be used herein. The various chemical procedures have their advantages and disadvantages including the complexity of the method involved, the ability to control the PEGylation of the selected molecule, the reversibility or degradability of the polyethylene glycol polymer linkage, the use of expensive or toxic solvents, etc.

PCT/GB95/00686 describes microspheres for biomedical uses which contain a substantially spherical core particle of a non-water soluble polymer and an outer surface consisting substantially of a water soluble polymer. The water soluble polymer is conjugated to polyethylene glycol and the non-water soluble core particle is attached to the water soluble polymer by the polyethylene glycol (PEG) moiety. The patent application describes detailed examples using PEG-dextran conjugates. Chitosan is also mentioned as a water soluble polymer in the preparation of such microspheres, but no PEG-chitosan conjugates were actually prepared. Moreover, there was no disclosure of particles in which the PEG moieties were pendant and exposed to the external environment.

Ouchi et al. describes the preparation of PEG-chitosan by acylating the amino groups on the polysaccharide with the monomethyl ether of PEG as the carboxylate and then covalently bonding this compound to 5-fluorouracil (5-Fu) to produce a material for cancer chemotherapy (Ouchi et al. J. Macromol. Sci. Chem., A28, 959, 1991). The active ester method was used to prepare the PEG-chitosan products in Ouchi et al and the chitosan was a low molecular weight material having a degree of polymerization of 30 which had been produced by acid treatment of chitosan. The 5-Fu attached to the end of the PEG chain in the PEG-chitosan conjugate and the resulting soluble complex was administered by injection.

Ouchi et al did not consider that an unmodified PEG-chitosan conjugate could be used for the delivery of anionic macromolecular drugs, such as antisense oligonucleotides and DNA (nucleic acids), nor did they consider that the unmodified conjugate could be used as a coating agent in the field of colloidal drug delivery through the interaction of the positively charged chitosan with negatively charged surfaces and negatively charged colloidal particles. Furthermore, there was no suggestion in Ouchi et al that such positively charged PEG-chitosan conjugates might be used to provide increased absorption of drugs across mucosal surfaces through their interaction with negatively charged mucus or negatively charged surfaces of epithelial cells.

In the field of gene therapy, it is often important to be able to compact a plasmid material comprising nucleic acids into a small particle for improved administration. The prior art describes how this may be accomplished using cationic polymers such as polylysine as well as cationic lipids (see for example Tomlinson and Rowland, J. Control. Rel. 39 357-372 (1995), Mumper et al., Pharm. Res. 13 701-709 (1996). A co-pending patent application PCT/GB97/00022 describes how such compaction can also be achieved using polyamidoamines to which polyethylene glycol has been attached.

However, no examples have been reported where PEG-chitosan was used for the improved compaction of plasmid DNA.

We have discovered that the modification of chitosan by the covalent bonding of a polyethylene glycol moiety to the chitosan molecule through the amino function (PEGylation) results in a polymer conjugate material that can display beneficial pharmaceutical properties, provides an opportunity for improvements in the delivery of drugs, vaccines, diagnostic agents as well as for nucleic acids in the field of gene therapy. In particular, the PEG-chitosan conjugate may be interacted with anionic materials, such as antisense oligonucleotides and DNA (nucleic acid), and may be used to enhance drug absorption or for targeting using particulate carriers.

According to a first aspect of the present invention there is provided a polymer conjugate comprising a chitosan moiety or a derivative thereof and a polyethylene glycol (PEG) moiety or a derivative thereof which are bonded together via the amino function on the chitosan by the use of an activated chitosan species.

According to a second aspect of the present invention there is provided a polymer conjugate comprising a chitosan moiety or a derivative thereof and a polyethylene glycol (PEG) moiety or a derivative thereof which are bonded together, the chitosan portion of the conjugate having a molecular weight in the range of from 10 kilodalton to 1000 kilodalton.

According to a third aspect of the present invention there is provided a composition comprising a complex formed between a PEG-chitosan conjugate and a therapeutic agent selected from the group consisting of oligonucleotides and nucleic acids. The PEG-chitosan conjugate comprises a chitosan moiety or a derivative thereof and a polyethylene glycol moiety or a derivative thereof which are bonded together.

According to yet another aspect of the present invention there is provided particular compositions comprising a polymer conjugate in the form of chitosan covalently attached to polyethylene glycol that can be used in drug delivery.

Chitosan is a polymeric material comprising repeating monomeric units having the formula: 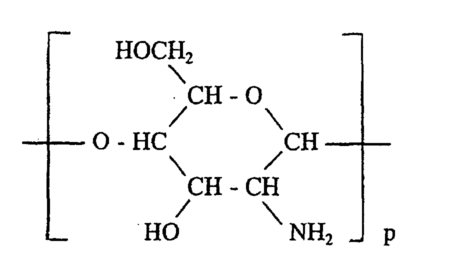 where p is an integer and represents the number of monomeric units in the chitosan chain, i.e. the degree of polymerisation.

In addition to the monomeric units shown above, chitosan will generally contain a proportion of the monomeric units found in chitin. This proportion will depend on the degree of deacetylation. Typically, the degree of deacetylation is in the range of from 99% to 10%, and is preferably in the range of from 90% to 20%, more preferably in the range of from 85 % to 40%.

It is possible to vary the degree of conversion of the chitosan to the PEGylated form by controlling the chemical reaction used to form the conjugate and so have a range of PEG-chitosan complexes for different pharmaceutical applications. The polyethylene glycol moiety may well increase the solubility of the PEG-chitosan conjugate or, more importantly, the solubility of a PEG-chitosan conjugate that has been complexed with a therapeutic agent such as a drug or a nucleic acid.

The conversion of the amino (NH₂) functions within chitosan to the PEGylated form can be within the range of 1 to 99%. However, it is preferred that the conversion is in the range 10 to 90% and more preferably in the range 10 to 50%.

When it is desired to use the PEG-chitosan conjugate to interact with a drug or a biological membrane or to modify the tight junction of a mucosal surface or to coat a negatively charged microparticle, then the PEG-chitosan conjugate should have a residual positive charge.

In one embodiment, the PEG-chitosan conjugate should retain sufficient positive charge to allow it to interact with negatively charged (anionic) materials such as oligonucleotides or nucleic acids (DNA) and provide for compaction of the anionic species. By compaction we mean reduction in particle size as measured by a technique such as atomic force microscopy or photon correlation spectroscopy.

In another embodiment, the PEG-chitosan conjugate should have sufficient positive charge to allow it to interact with negatively charged colloidal carriers intended for drug delivery, such as emulsions, liposomes, microspheres and microcapsules as well as negatively charged drug particles of a size less than 100 microns.

In yet another embodiment, the PEG-chitosan conjugate should have a sufficient positive charge to interact with mucin or epithelial cells.

A positive charge on the PEG-chitosan conjugate can be evaluated by studying the interaction of the conjugate with a negatively charged polymer so that a polyelectrolyte complex is formed as described by Terayama in J. Polymer Science, 8 243 (1952). Alternatively, the PEG-chitosan conjugate can be absorbed onto a polymer particle such as a polystyrene microsphere of about 1 micron in size and the charge on the system evaluated by particle microelectrophoresis using an apparatus such as the Malvern Zeta Sizer. By a sufficient positive charge for interacting with the above described anionic species, we typically mean a charge, measured as the Zeta potential, of at least 1mV and preferably in the range of from 1 to 200 mV at pH 7.4 in 1mM HEPES buffer. In order that the PEG-chitosan conjugate has sufficient positive charge, not more than 90% of the amine groups in chitosan should be PEGylated.

The person skilled in the art will be able to ascertain the degree of conversion necessary to meet the required pharmaceutical application.

The chitosans used for the preparation of the PEG-chitosan conjugates can have a molecular weight in the range of from 10 kD to 1000 kD, but more preferably will have a molecular weight in the range of from 10 kD to 500 kD, e.g. 20 kD to 500 kD, particularly from 30 kD to 300 kD, e.g. between 100 kD and 300 kD.

The degree of polymerisation of the chitosans used in the preparation of the PEG-chitosan conjugates is typically in the range of from 50 to 6000, preferably in the range of from 100 to 3000 and particularly in the range of from 150 to 2000.

The polyethylene glycol content of the PEG-chitosan conjugate is typically from 1 to 50% on a weight basis, preferably from 5 to 20%.

Chitosans that have been derivatised by modification of the hydroxyl function could also be used. Such derivatives include O-acylated and alkylated materials such as O-benzoyl chitosan and O-sulphated chitosans as detailed in Chitin Chemistry, A F Roberts, Macmillan, 1992, p. 166. Thus, in the present invention the chitosan moiety in the PEG-chitosan conjugate may be a chitosan derivative and by the term chitosan we are also intending to include derivatives thereof. Preferably, however, the chitosan is unmodified.

The PEG-chitosan conjugate can be used to modify surfaces that are negatively charged or to interact with or form complexes with negatively charged molecules such as DNA, DNA plasmids or nucleic acids. The positively charged chitosan can bind strongly to such a negative surface and in doing so will expose the PEG group to the external environment. Such an exposed polyethylene glycol group will tend to provide for steric stabilization. This can lead to an improved stability, e.g. for a colloidal dispersion, but can also have important biological implications in minimising the uptake of proteins to the surface of a particle, e.g. when the particle is injected into the bloodstream or administered to a body compartment.

The PEG-chitosan conjugate or adduct may be prepared using techniques known in the art for bonding PEG moieties to polymeric materials.

In one embodiment, the PEG-chitosan conjugate is prepared by a process comprising the steps of:
(1) preparing an activated PEG or an activated PEG derivative by incorporating an acrylic ester, an acrylic thioester or an acrylamido group into the PEG or PEG derivative;
(2) reacting the activated PEG or PEG derivative with chitosan; and
(3) recovering the conjugate of the PEG or PEG derivative and chitosan.

This process allows the reaction between the PEG or PEG derivative and chitosan to be controlled.

By a PEG derivative we mean a modified polyethylene glycol, for example polyethylene glycol in which one or both of the terminal hydroxyl groups has been previously modified. Suitable PEG derivatives include alkoxy PEGs in which a terminal hydroxyl group(s) has been converted into an alkoxy group, i.e. a group having the formula RO- in which R is alkyl. Preferred PEG derivatives are those comprising a single alkoxy terminal group. Preferred alkoxy groups are C₁₋₄ alkoxy, such as methoxy.

The preparation of a PEG or PEG derivative carrying an acrylic ester group can be carried out by reacting a PEG or PEG derivative containing a hydroxyl function with acryloyl chloride.

The preparation of a PEG or PEG derivative carrying an acrylic thioester group can be carried out by reacting a PEG or PEG derivative containing a sulfhydryl group with acryloyl chloride. The preparation of a PEG or PEG derivative carrying an acrylamido group can be performed by reacting PEG or a PEG derivative containing one primary or secondary amine function with acryloyl chloride. The activated PEG or PEG derivative can be characterized by Gel Permeation Chromatography (GPC), FT-IR and UV spectroscopy. The degree of activation can be determined by end-group titration which involves adding an excess of 2-mercaptoethanol and titrating excess thiol with a calibrated KI/I₂ solution, or by UV spectroscopy, after calibration with a standard acrylamide or acrylic ester such as N-acryloylmorpholine or tetraethyleneglycol diacrylate, conducted at 233 nm.

Activated PEGs or activated PEG derivatives can be stored for several months at T ≤ 4°C in the presence of a desiccant. A radical inhibitor such as 4-methoxyphenol may also be added to prevent radical polymerization.

The reaction of the activated PEG or activated PEG derivative with chitosan is preferably performed in an aqueous media. Alcohols or alcohol/water mixtures can also be used. The pH of the reaction medium is preferably at least 8 and typically in the range 8 to 9. The preferred reaction temperature is between 20 and 30°C and typical reaction times are 12-48 hours. The occurrence of vinyl radical polymerization is inhibited by conducting the reaction under inert atmosphere, in the dark and in the presence of a radical inhibitor such as 4-methoxyphenol. The molecular weight of the PEG or the PEG portion in the case of a PEG derivative is typically between 1 and 30 kD, preferably between 2 and 20 kD and more preferably between 2 and 10 kD.

Recovery of the PEG-chitosan conjugate or adduct is generally carried out by ultrafiltration in water or by evaporating the reaction solvent and extracting the crude material with a new solvent.

The purity of the adduct can be assessed by GPC, FT-IR, NMR and UV spectroscopy. Checking for residual activated PEG or activated PEG derivative is usually performed spectrophotometrically at 233 nm.

The method can have advantages over other PEGylation processes known in the art because:
(a) free base is the species reacting with the double bond so that the reaction rate tends to increase with increase in pH, i.e. with increasing the concentration of free base;
(b) no by-product is produced during the reaction; and
(c) the aminic character of the amino groups on the chitosan is not changed as a consequence of the grafting reaction.

In another embodiment, the PEG-chitosan conjugate is prepared by a process which involves activating the chitosan rather than the PEG or PEG derivative. Activation of the chitosan is preferably achieved by adding a large excess of a bis(acrylamide) to the chitosan. After the chitosan has reacted with the bis(acrylamide), the excess bis(acrylamide) is preferably removed and the activated chitosan reacted with a PEG or PEG derivative, preferably a monofunctional PEG carrying a hydroxyl, sulfhydryl or primary or secondary amino group.

Accordingly, in a further aspect of the present invention there is provided a process for preparing a PEG-chitosan conjugate comprising the steps of:
(1) preparing an activated chitosan by reacting it with a bis(acrylamide);
(2) optionally removing any excess bis(acrylamide),
(3) reacting the activated chitosan with a PEG or PEG derivative, preferably a monofunctional PEG carrying a hydroxyl, sulfhydryl or primary or secondary amino group; and
(4) recovering the conjugate of the monofunctional PEG and chitosan.

Grafting bis(acrylamide) to chitosan may make the chitosan more soluble or at least more swellable at pH ≥ 8 and, moreover, may provide for reactive functions which are more accessible than the original NH₂ groups which are close to the main polymer chain.

Furthermore, activating the chitosan with bis(acrylamide) may allow higher amounts of PEG to be grafted or bonded to the chitosan.

The PEG-chitosan conjugates prepared by the above described methods comprise a chitosan moiety and a PEG moiety which are joined together through a linking group or coupling moiety having the formula -CH₂CH₂C(O)-R²-X-. The linking group is bonded to the amino nitrogen on the chitosan moiety via the -CH₂CH₂- group and to the PEG moiety via the X group. In the linking group, R² is a bond or a divalent organic group, X is oxygen, sulphur or -NR³- and R³ is H or an organic group such as alkyl, e.g. C₁₋₁₀ alkyl.

Accordingly, in a further aspect of the present invention there is provided a PEG-chitosan conjugate having the formula:

-[Cm-NR¹₂]ₚ- I

wherein:
Cm is the remainder of the monomeric unit making up the chitosan moiety or a derivative of such a monomeric unit;
p is an integer and represents the number of monomeric units of formula -Cm-NR¹₂- in the chitosan moiety,
each R' is independently H or a group -A-PEG providing that at least a proportion of the R¹ groups are -A-PEG;
each PEG is independently a polyethylene glycol moiety or a derivative thereof;
each A is independently a linking group having the formula -CH₂CH₂-C(O)-R²-X- which is bonded to the amino nitrogen on the chitosan moiety via the -CH₂CH₂- group and to the PEG moiety via the X group;
R² is a bond or a divalent organic group;
X is oxygen, sulphur or -NR³-; and
R³ is H or an organic group,
or a physiologically acceptable derivative thereof.

### In the above Formula I:

For the avoidance of doubt, in referring to Cm as the remainder of the monomeric unit making up the chitosan moiety we are referring to a group which together with the -NH₂ group makes up the full monomeric unit found in chitosan. Thus, the complete monomeric unit itself has the formula Cm-NH₂. By a derivative of such a monomeric unit we are referring to physiologically acceptable derivatives in which a hydroxyl group has been modified. Suitable derivatives include O-acylated and alkylated materials such as O-benzoyl chitosan and O-sulphated chitosans as detailed in Chitin Chemistry, A F Roberts, Macmillan, 1992, p. 166.

Cm is preferably the remainder of the monomeric unit making up chitosan.

p is preferably an integer in the range of from 50 to 6000, more preferably in the range of from 100 to 3000 and particularly in the range of from 150 to 2000.

PEG is preferably a modified polyethylene glycol moiety having the formula 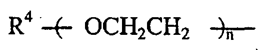 where R⁴ is an aliphatic, alicyclic or aromatic hydrocarbon group, such as an alkyl, cycloalkyl, aryl or aralkyl group, and n is an integer. Preferably R⁴ is alkyl, more preferably C₁₋₁₀ alkyl, e.g. C₁₋₄ alkyl, and especially methyl. n is suitably in the range of from 10 to 1000 and is preferably in the range of from 10 to 100.

R² is preferably a bond or a divalent organic group having the formula: 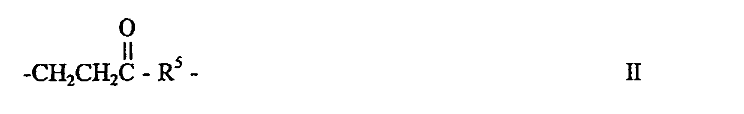 where R⁵ is a divalent organic group which is bonded to the carbonyl (C(O)) group of linking group A. Preferably R⁵ is bonded to the carbonyl group in Formula II above and the carbonyl group of linking group A via nitrogen atoms. More preferably, R⁵ is a group having the formula: 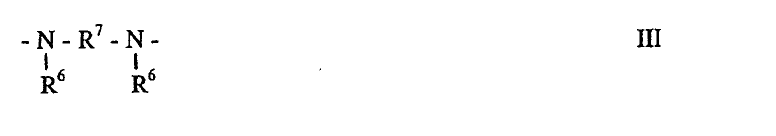 or 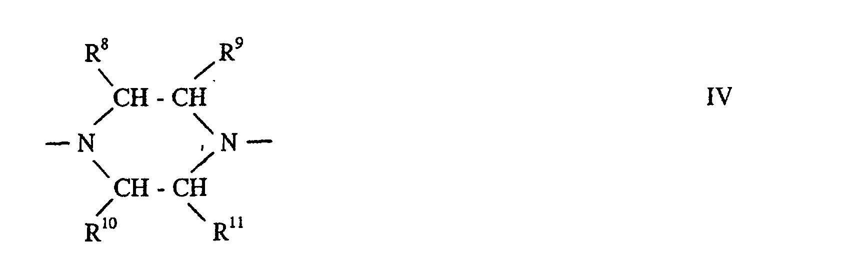 where each R⁶ is independently a linear or branched C₁₋₄ alkyl chain, R⁷ is a divalent organic group, particularly a linear or branched C₁₋₄ alkylene chain and R⁸, R⁹, R¹⁰ and R¹¹ are each independently H or a linear or branched C₁₋₃ alkyl chain.

X is preferably oxygen or -NR³-.

Suitable organic groups for R³ include hydrocarbon groups such as alkyl, e.g. C₁₋₁₀ alkyl. Preferably R³ is H.

The structure of the PEG-chitosan conjugates resulting from the two methods described above is somewhat different. For example, if we employ an aminated monomethoxy-PEG having the structure: 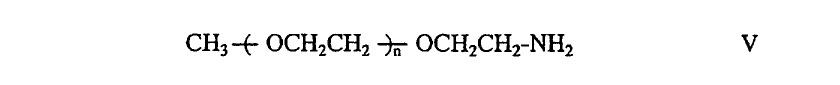 and if chitosan is represented as: 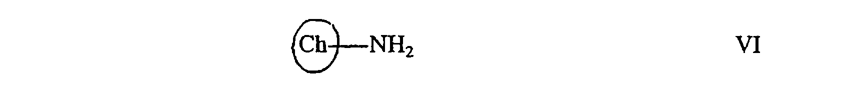 where 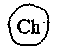 represents the polymeric chain in chitosan and -NH₂ represents a single primary amine group in the chain, then the conjugate prepared by activating the aminated monomethoxy-PEG with an acrylamido group and reacting the activated compound with chitosan has the structure:

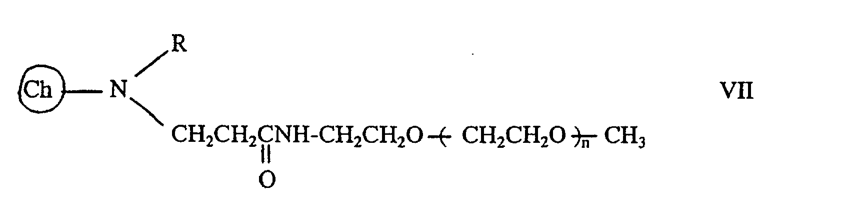

In contrast, if the alternative process is employed in which the chitosan is activated by reacting it with a bis(acrylamide) having the structure: 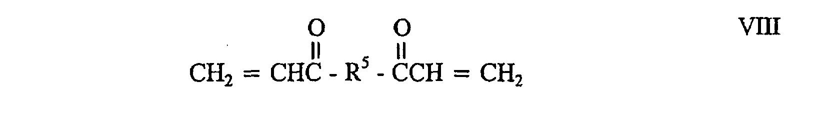 where R⁵ is a group having the formula: 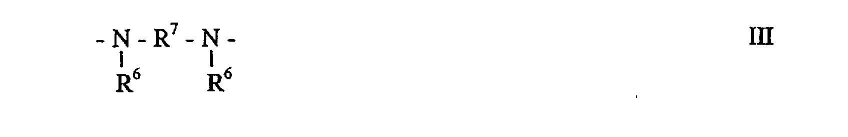 or 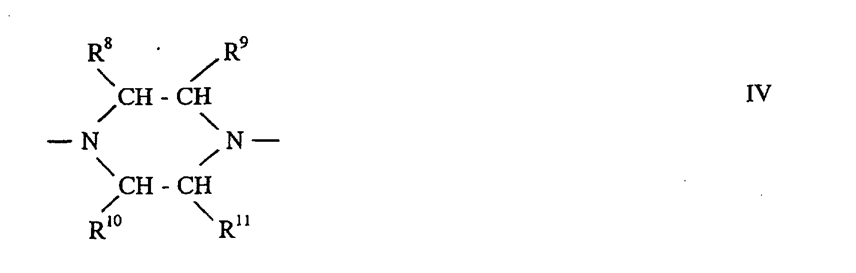 the corresponding conjugate has the following structure: 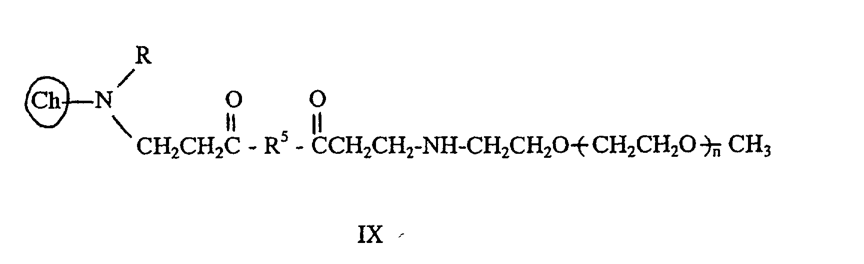 wherein:
R is hydrogen or a second PEG chain bound to chitosan in the same way, that is through an acrylamide (method 1) or a bis(acrylamide) (method 2) coupling moiety;
R⁶ is a linear or branched C₁₋₄ alkyl chain;
R⁷ is a linear or branched C₁₋₄ alkylene chain; and
R⁸, R⁹, R¹⁰ and R¹¹ are each independently H or a linear or branched C₁₋₃ alkyl chain.

In drug targeting and drug delivery, as well as in diagnostic imaging and in the administration of vaccine systems, particulate materials in the form of emulsions, liposomes, microparticles, microcapsules and nanoparticles are employed. Such particles often carry a net negative charge. It is often important to be able to modify this negative charge through the process of surface modification. For example, as mentioned above, the properties of liposomes can be modified by the adsorption of chitosan. It is now possible through the present invention to modify such particles with the PEG-chitosan system so that the particle carries not only a positive charge but a polyethylene glycol group that will provide for steric stabilisation and beneficial biological and physical properties.

In the field of gene therapy, it is often important to be able to compact a plasmid material comprising nucleic acids into a small particle for improved administration. We have discovered that a PEG-chitosan conjugate can be used to compact plasmid DNA. The compacts produced should have enhanced solubility and beneficial properties for the interaction with cells and the subsequent expression of the gene product.

The PEG-chitosan material described herein could be further modified by the attachment of targeting ligands to the hydroxyl and unmodified amino functions in order to achieve site specific targeting. Such ligands can take the form of sugars and proteins. The latter can include monoclonal antibodies and fragments thereof. The sugars include mannose, fucose and galactose. The choice of sugar will be dictated by the nature of the tissue or cell that is identified as the target site. For example, a complex of PEG-chitosan with DNA could be targeted to the liver by the covalent attachment of a triantennary galactose moiety.

Figure 1 is a graph showing the effect of added Na₂SO₄ on the properties of polystyrene particles (190 nm) coated with chitosan or PEG-chitosan.

Figure 2 is a graph showing the effect of increasing chitosan and PEG-chitosan concentrations on the size of the complexes formed between these materials and DNA.

Figure 3 is a graph showing the effect of adding chitosan and PEG-chitosan on the fluorescence of a DNA/ethidium bromide complex.

The present invention is now illustrated but not limited with reference to the following examples.

### Example 1 Preparation of PEG-chitosan using activated PEG

### (A) Preparation of monomethoxy-PEG piperazinyl formate (MPEG-PP)

Monomethoxy-PEG 1900 (12.26 g, 6.45 mmol) was dissolved in "alcohol-free" CHCl₃ (60 ml). The solution was dried overnight over calcium hydride, which was then removed by filtration. 1,1'-carbonyldiimidazole of 97 % purity (2.16 g, 12.9 mmol) was then added and the resulting solution allowed to stand at 30°C for 30 min after which cold water was added (10 ml) and the mixture stirred for 10 min. After separation of the phases, anhydrous piperazine (0.56 g, 12.9 mmol) was added to the organic phase and allowed to react for 20 hours at 25°C. The solution was then diluted with CHCl₃ (100 ml), extracted with water (5 x 30 ml), dried with Na₂SO₄, filtered, concentrated in vacuo to 60 ml and finally poured into diethyl ether at about 10°C. The powder which precipitated out was collected by filtration and dried to constant weight at 0.1 torr. The yield was 10.64 g. The GPC chromatogram exhibited one peak with retention time 1030 sec. The FT-IR spectrum showed a band in the urethane region at 1703 cm-¹. The molecular weight, determined by potentiometric titration with 0.1 M HCl, was 2190.

### (B) Preparation of monomethoxy-PEG acrylamide (MPEG-AA)

MPEG-PF (7.07 g; 3.23 mmol) was dissolved in dry, "alcohol-free" CHCl₃ (10 ml) and distilled water (2 ml) was added. 2.4 ml of a 2M solution of acryloyl chloride in CHCl₃ (4.8 mmol) and 2.4 ml of a 2M NaOH aqueous solution (4.8 mmol) were then added together, drop-wise, under vigorous stirring while maintaining the temperature at about 5°C. Once the addition was completed, the reaction mixture was left at 25°C in the dark and under stirring for 1 hour. The reaction mixture was then diluted with CHCl₃ to 150 ml and washed in a separatory funnel with 5% KNO₃ (2 x 30 ml) and water (1 x 30 ml). After drying with Na₂SO₄ and filtering, the volume was reduced to about 50 ml by evaporating most of the CHCl₃ *in vacuo*. The solution was then diluted with diethyl ether (200 ml). The product which precipitated out was collected by filtration and dried to constant weight at 0.1 torr. The yield was 6.05 g. The GPC chromatogram exhibited one peak with retention time 1020 sec. The FT-IR spectrum was very similar to that of starting MPEG-PF, but showed an amidic band at 1649 cm⁻¹. The molecular weight, determined spectrophotometrically at 233 nm in water using the calibration curve of N-acryloylmorpholine, was 2300.

### (C) PEGylation of chitosan

Chitosan hydrochloric salt (SeaCure CL 113) (1.03 g, 5.2 meq), MPEG-AA (3.91 g, 1.7 mmol) and 4-methoxyphenol (5 mg) were dissolved in distilled water and the pH of this mixture was raised to 8 by adding drop wise and under vigorous stirring triethylamine dissolved in a 3:1 H₂O/CH₃OH mixture. A precipitate was formed at pH>7. The reaction mixture was stirred at 25°C under a nitrogen atmosphere and in the dark for ten days. The pH was brought to 3-4 by addition of diluted HCl and the resulting solution was freeze-dried. The powder was collected, extracted in a Soxhlet apparatus with a 3:1 diethyl ether/CH₂Cl₂ mixture and dried in vacuo up to constant weight. The yield was 1.20 g.

The UV spectrum recorded in water did not exhibit any peak at 233 nm indicating that no residual MPEG-AA was present. In the FT-IR spectrum a shoulder at around 1700 cm⁻¹ was detected which was absent from the spectrum for native chitosan so indicating the presence of grafted PEG. Elemental analyses performed on the product (C: 37.69%, H: 7.17%, N: 5.78%, Cl: 14.09%), on starting chitosan (C: 34.91%, H: 6.42%, N-6.56%, Cl: 17.04%) and on MPEG-AA (C: 54.49 %, H: 8.87%, N: 1.25%) established that the PEG content was 14% by weight. Mohr titration of chloride ions executed on both the product and native chitosan confirmed this value.

The same process as described above has been used to prepare PEG-chitosan complexes from chitosans having molecular weights in the range 10,000 to 500,000 Daltons and from polyethylene glycol materials having molecular weights in the range 750 to 10,000 Daltons. The degree of conversion of the chitosan to the PEGylated form can be controlled through the chemical reaction.

In the above method for preparing the PEG-chitosan conjugate, it could be beneficial to increase the solubility of the chitosan at pH > 7, and we have found that isobutyric acid gives a more soluble chitosan salt at about pH 7 compared to hydrochloric acid.

### Example 2 Preparation of PEG chitosan using activated chitosan

### (A) Preparation of monomethoxy-PEG piperazinyl formate (MPEG-PF)

Monomethoxy-PEG piperazinyl formate (MPEG-PF) was prepared using exactly the same technique as described in Example 1 (part A) above, but using monomethoxy-PEG 550 (3.55 g, 6.45 mmol) instead of monomethoxy-PEG 1900. The yield was 5.18 g.

### (B) PEGylation of chitosan

Chitosan (Fluka; MW = 70000) (0.40g, 2.5 meq) was dissolved in distilled water (30 mls) containing isobutyric acid (0.22 g, 2.5 meq). 1,4-bis(acryloylpiperazine) (2.43 g, 12.5 mmol) was then added to the chitosan solution and allowed to react for 24 hours at 25°C in the dark and under a nitrogen atmosphere. Unreacted 1,4-bis(acryloylpiperazine) was then removed by extraction with chloroform (8 x 5 ml). MPEG-PF was then added (3.25 g, 5.0 meq) to the aqueous phase and allowed to react for 10 days at 25°C in the dark and under a nitrogen atmosphere, the pH being adjusted to 7 by the addition of isobutyric acid. The solution was freeze dried, extracted in a Soxhlet apparatus with a 3:1 diethyl ether/CH₂Cl₂ mixture and dried in vacuo to constant weight. The yield was 0.38 g.

Grafting of the PEG onto the chitosan was confirmed by FT-IR spectroscopy with a peak being observed at 1700 cm⁻¹ which is typical of PEG-piperazinyl formate and absent in native chitosan. From the ratio of the area of this peak to that at 1630 cm-1 (which is typical of chitosan), the PEG content was calculated to be around 20 % by weight.

### Example 3 The physical adsorption of PEG-chitosan to colloidal particles

To illustrate the physical adsorption of PEG-chitosan to particles, polystyrene nanoparticles were used as a model system. The polystyrene particles were purchased from IDC Spheres, Interfacial Dynamics Corp. USA. The particles were stabilised with negative sulphate groups. The particle size of the unmodified lattices, as measured by photon correlation spectroscopy (PCS) (Malvern S4700, UK), was 190 nm.

Samples of the polystyrene particles were coated with either chitosan (SeaCure CL 113) or with a PEG-chitosan conjugate. The PEG-chitosan conjugate was prepared as in Example 1 from chitosan SeaCure CL 113 obtained from Pronova Ltd., Norway. The chitosan had an average molecular weight of about 50 kD and an intrinsic viscosity of 153 ml/g. About 12% of the amino functions were acetylated (88 % deacetylated). The average molecular weight of the attached PEG was 2100 and the degree of PEGylation 15%.

Different quantities of the chitosan and PEG-chitosan conjugate were added to 10 ml glass test tubes (0-100 µg) together with 1.5 ml of purified water. 0.5 ml of a 0.1% suspension of polystyrene particles was then added (representing 500 µg of particles) drop wise with vigorous stirring for 2 hours. The effect of the adsorbed chitosan and PEG-chitosan on the zeta potential of the particles was measured using a Malvern Zeta Sizer (Mark IV), Malvern Instruments, UK, at pH 7.4 in 1 mM HEPES buffer.

The uncoated polystyrene particles carried a net negative charge of -50 mV. The addition of chitosan or PEG-chitosan gave rise to a rapid charge reversal as measured using a Zeta Sizer so that the polystyrene particles carried a net positive charge of +20 mV upon the addition of 100 µg of chitosan or PEG-chitosan to 500 µg of the polystyrene particles suspended in 2 ml of an aqueous buffer solution. Hence, both chitosan and PEG-chitosan have the ability to strongly adsorb to a negatively charged colloidal particle and reverse the charge. The point of zero charge was reached after the addition of 12 µg of chitosan to the system and 18 µg of PEG-chitosan to the system, demonstrating the difference between the PEGylated and unPEGylated materials. A similar difference could be seen for the addition of the same quantity of chitosan or PEG-chitosan to the system. For example, for 20 µg added cationic polymer the zeta potential was + 13 mV for the chitosan system and +5 mV for the PEG-chitosan system.

The addition of a positively charged polymer to a negatively charged colloid such as polystyrene particles of 190 nm will give rise to flocculation. This process was investigated by measuring the optical density (OD) of the system at 600 nm after dilution 50:50 with the dispersion medium before measurement. For the addition of chitosan, the concentration of cationic polymer required to obtain a maximum OD of 0.68 was 5 µg (added to 500 µg of polystyrene particles in 4 ml). For the PEG-chitosan system, a maximum OD of 0.66 was obtained at 10 µg of PEG-chitosan added to 500 µg polystyrene particles suspended in 4 ml. Thus, both chitosan and PEG-chitosan cause flocculation of negatively charged model colloidal particles, but the quantities required were different so reflecting the difference between PEG-chitosan and chitosan.

The ability of coated particles to withstand flocculation was measured by studying the effect of added sodium sulphate (Na₂SO₄). The Na₂SO₄ induced flocculation of coated polystyrene particles (190 nm) was measured using a weight ratio of chitosan or PEG-chitosan to particles of 1:5. The optical density (OD) at 600 nm was used to assess the properties of the coated particles.

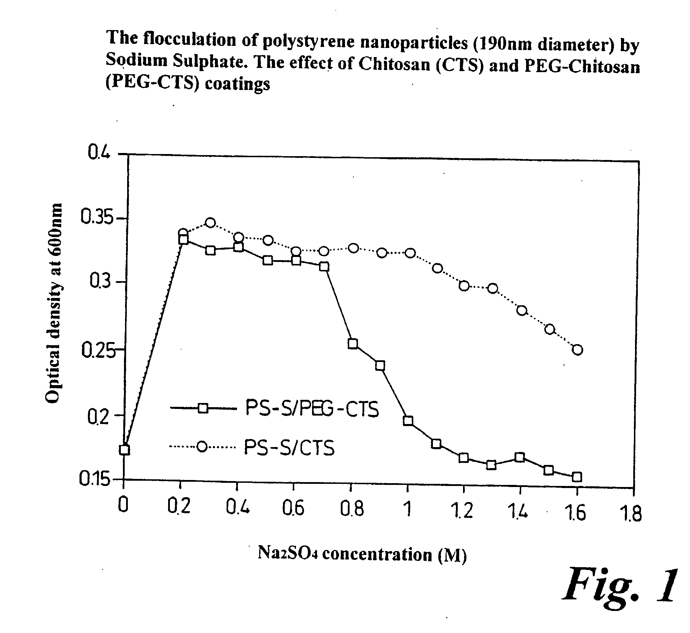

Figure 1 shows the effect of added Na₂SO₄ on the properties of polystyrene particles (190 nm) coated with chitosan and PEG-chitosan, respectively. (0.2 ml of polymer coated particles (10 µg chitosan or PEG-chitosan/50 µg polystyrene)) in 0-1.6 M Na₂SO₄ solution). Both the chitosan and PEG-chitosan coated particles were affected by low concentrations of Na₂SO₄ (the uncoated particles required at least 0.25 M Na₂SO₄ to commence a change in OD). The OD of the chitosan coated particles remained constant at Na₂SO₄ concentrations above 0.8 M Na₂SO₄ while the PEG-chitosan coated particles demonstrated a change in OD.

These results again demonstrate the difference between chitosan and PEG-chitosan as coating materials.

### Example 4 Flocculation of chitosan and PEG-chitosan

The different properties of chitosan and PEG-chitosan can be measured by the Na₂SO₄ induced flocculation of solutions of the two cationic polymers. Two different quantities of the chitosan and PEG-chitosan as described in Example 3 were suspended in 0.4 ml of water, 0.1 mg and 1 mg. Then Na₂SO₄ was added to induce flocculation. The degree of flocculation was measured as previously as the OD at 600 nm. At an Na₂SO₄ concentration of 0.2 M the following OD figures were obtained.

| | |
|---|---|
| PEG-chitosan (0.1 mg) | OD = 0.005 |
| Chitosan (0.1 mg) | OD = 0.02 |
| PEG-chitosan (1 mg) | OD = 0.045 |
| Chitosan (1 mg) | OD = 0.13 |

The results show that for the same concentration of cationic polymer, the PEG-chitosan system is more stable to Na₂SO₄ induced flocculation than chitosan.

### Example 5 Compaction of DNA with PEG-chitosan - Physicochemical Studies

In order to demonstrate the difference between the compaction of DNA with chitosan and the compaction of DNA with the PEG-chitosan conjugate, a model plasmid material was employed. This is in the form of the plasmid CMV-CAT that encodes for chloramphenicol acetyl transferase (CAT), a so-called reporter gene system. The p-CAT material was obtained from Gene Medicine Inc., Houston, USA.

The interaction between a plasmid and a cationic polymer can be followed by a variety of techniques including the following:
(1) Particle size measurement on the complex using photon correlation spectroscopy.
(2) Measuring the zeta potential of the plasmid-complexing agent complex using particle microelectrophoresis.
(3) Measuring the state of compaction of the plasmid by studying the fluorescence of the plasmid DNA material in the presence of ethidium bromide.
(4) Titration microcalorimetry in which the heat of interaction between the positively charged polymer conjugate and the negatively charged DNA material is determined.

In this example and in Example 6 which follows, a number of the above techniques were used to illustrate the differences between compaction using chitosan and compaction with the PEG-chitosan system. The PEG-chitosan was prepared from a sample of SeaCure CL 113, where about 12% of the amino functions were acetylated. The method described in Example 1 was employed. The average molecular weight of the PEG bound to the chitosan was 2100. Unmodified chitosan SeaCure CL 113 was used as a control.

Photon Correlation Spectroscopy and Zeta Potential Measurements:
The pCAT-DNA/chitosan or the pCAT-DNA/PEG-chitosan complexes were prepared in the following way to give different pCAT-DNA/polymer ratios.

81.1 µg (100 µl) of pCAT-DNA in 2 ml of ultra pure water was slowly mixed with 525 µg of chitosan or PEG-chitosan (0.35 ml of a 0.15 % solution) under stirring to give a pCAT-DNA:polymer weight ratio of 1:6.5.

81.1 µg (100 µl) of pCAT-DNA in 2 ml of ultra pure water was rapidly mixed with 810 µg of chitosan or PEG-chitosan (0.81 ml of a 0.1 % solution) under stirring to give a pCAT-DNA:polymer weight ratio of 1:10

The size of chitosan and PEG-chitosan complexes and their zeta potentials in water and 1 mM HEPES buffer at pH 7.4 were determined using a Malvern S4700 PCS and a Malvern Zeta Sizer (Mark IV), respectively. It can be seen from Table 1 that the surface properties of the chitosan-DNA complex are different from the properties of the DNA complex made with the conjugate that is the subject of the present invention. It is believed that the improved solubility of this complex and the difference in particle charge is beneficial for a biological response. Interestingly, the complexes formed by chitosan and PEG-chitosan with DNA produced small sized nanoparticles of about 140 nm as measured by PCS.

It will be appreciated by those skilled in the art that the size of the resulting complexes between plasmid DNA and a compacting polymer will be affected by the ratio of the interacting components and the processing conditions (i.e. mixing speed).

**Table 1**

| **Particle size and zeta potential of chitosan (PEG- chitosan)/DNA complexes** | | | | |
|---|---|---|---|---|
| Ratio (w/w) (µg/µg) | DNA/CTS | | DNA/PEG-CTS | |
| | Size (nm) | ZP (mV) | Size (nm) | ZP (mV) |
| 1:6.5 | 138.3 | 32.2 | --- | 20.9 |
| 1:10 | 172.5 | 40.4 | 140.8 | 31.2 |

### Example 6

In this example, a more detailed study of the interaction between a selected plasmid DNA material (CMV-CAT) and PEG-chitosan was carried out. The plasmid was at a concentration of 1 mg/ml and the PEG-chitosan at 500 microgram/ml. The PEG-chitosan was centrifuged at 10,000 rpm for 10 mins to remove any particulate from the solution. To a solution of 50 µg of DNA in 1 ml of water, was added an aliquot of PEG-chitosan solution equivalent to charge ratio DNA:PEG-chitosan of 1:0.25. The solution was stirred for 5 minutes and left to complex for 10 minutes.

The size of the complex was determined using Photon Correlation Spectroscopy. Further aliquots of PEG-chitosan were then added.

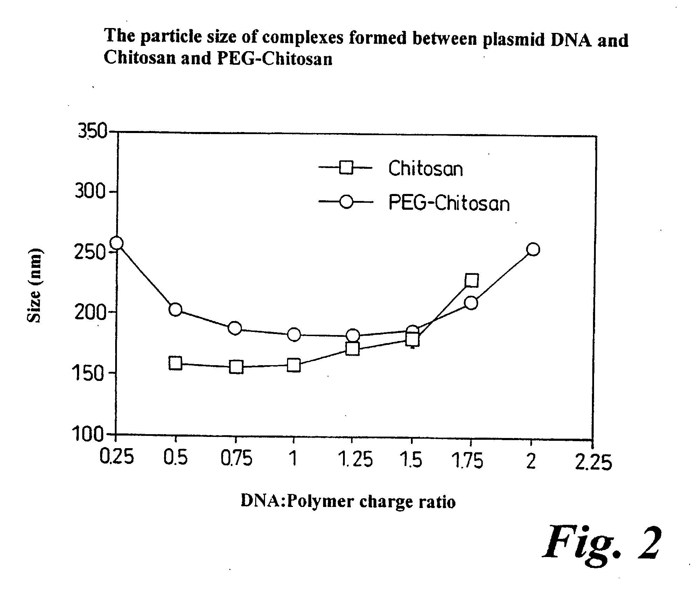

Figure 2 shows the effect of increasing chitosan and PEG-chitosan concentrations on the size of the resultant complexes. At low chitosan or PEG-chitosan concentration, there is not sufficient polymer to condense the plasmid DNA. However, at a ratio between 0.75 - 1.5 of DNA:PEG-chitosan or DNA-chitosan, a complex is formed which is reduced in size to less than 190 nm at a charge ratio close to 1:1. With further increase in polymer concentration, the size of the complex increases. The results show the importance of the amount of PEG-chitosan added for complexing DNA with the polymer. The results also show that the presence of the PEG function does not greatly affect the condensing properties of chitosan. The greater size of the PEG chitosan complexes as compared to the chitosan complexes is considered to be due to the PEG group providing a steric barrier.

### Fluorescence Study:

The interaction of DNA with chitosan and with the PEG-chitosan conjugate was examined through a process of fluorescence determination using ethidium bromide (EtBr) as a marker material. Plasmid DNA interacts (intercalates) strongly with the ethidium bromide to display strong fluorescence. However, if the plasmid is compacted then the fluorescence decreases markedly suggesting displacement.

The decrease in fluorescence was measured as follows.

8.11 µg of plasmid DNA was mixed with 2 µg of EtBr. The fluorescence of this complex was determined using a fluorescence spectrometer (Perkin-Elmer 3000, UK) with emission at 591 nm and excitation at 366 nm. 1 µg portions of chitosan or PEG-chitosan were added incrementally to the complex and the decreased fluorescence of the complex measured. Chitosan (CL 113) and PEG-chitosan were used to compare the efficiency of EtBr displacement.

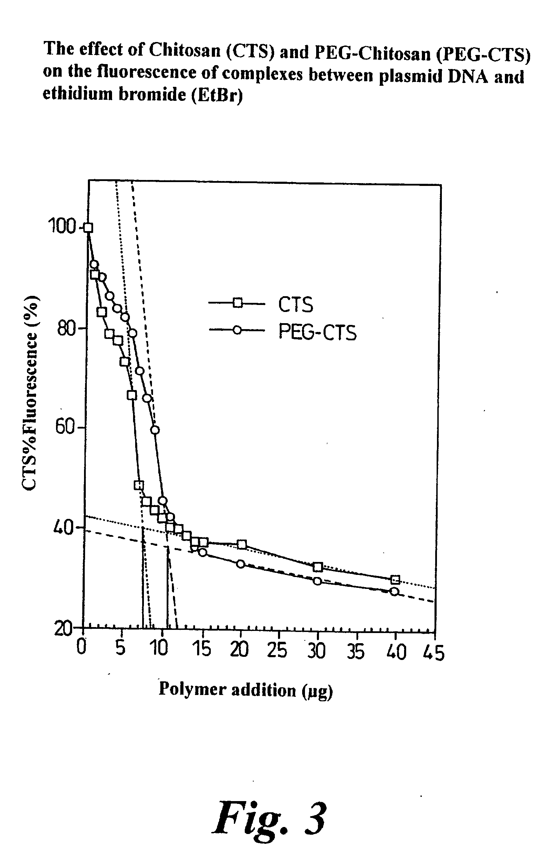

The displacement reaction is shown in Figure 3. As seen in Figure 3, the compaction of the selected plasmid DNA material with chitosan displays a different profile to the compaction with PEG-chitosan.

For chitosan (CTS) the displacement reaction finished when the addition of chitosan calculated from Figure 3 was 7.47 µg. For PEG-chitosan (PEG-CTS) this value was 10.66 µg. At this point, the weight ratio of DNA:chitosan was 1:0.92 and the weight ratio of DNA:PEG-chitosan 1.31.

Although more PEG-chitosan than chitosan was added to finish the displacement reaction on a weight basis, the molar concentrations of the two polymers were very close - 0.033 µmol for chitosan and 0.039 µmol for PEG-chitosan.

The example shows that the PEGylation of chitosan does not substantially affect the ability of chitosan to intereact strongly with plasmid DNA.

### Microcalorimetry:

The interaction between chitosan and PEG-chitosan and a negatively charged plasmid was assessed by the heat exchange that occurred during the reaction using a technique known as isothermal titration microcalorimetry. A thermal activity monitor available from Thermometric, Model 2277 in Sweden was used to monitor the heat change.

The method involved injecting a solution of chitosan or PEG-chitosan from a precision syringe into two DNA solutions at specified time intervals. 2.0 ml of a DNA solution containing 81.1 µg pDNA was used for each experiment and 0.35 ml of the chitosan and PEG-chitosan solutions were added as 35 separate 10 µl injections. The time interval between two injections was about 10 minutes.

Each injection of chitosan or PEG-chitosan led to the evolution (exothermic process) or absorption (endothermic process) of heat which was picked up by the thermal activity monitor and recorded as a series of peaks, the value of which was calculated. The heat change was measured after each addition.

0.35 ml of the chitosan and PEG-chitosan solutions were also added to 2.0 ml distilled water as 35 separate 10 µl injections in order to determine the heat of dilution which was then subtracted from the measured heat exchange resulting from the interaction between chitosan and PEG-chitosan and the DNA solution.

Initially the interaction between the DNA and both the chitosan and PEG-chitosan was an exothermic process which became less exothermic as more of the chitosan and PEG-chitosan solutions were added, eventually becoming endothermic.

The interaction was followed by measuring the quantity of chitosan or PEG-chitosan needed to provide an endothermic response. The ratio of DNA to chitosan or PEG-chitosan at this point was then calculated (Table 2).

**Table 2**

| **The interaction between chitosan or PEG-chitosan and Plasmid DNA** | |
|---|---|
| CTS or PEG-CTS | DNA/CTS or PEG-CTS ratio (w/w) |
| 0.05% CTS | 1:0.80 |
| 0.12% CTS | 1:0.74 |
| 0.15% CTS | 1:0.74 |
| 0.15% PEG-CTS | 1:1.11 |
| 0.20% PEG-CTS | 1:0.99 |
| 0.30% PEG-CTS | 1:1.11 |

The stoichiometry of the interaction can be calculated:

The mean MW of plasmid DNA per a phosphate group was calculated as 308.8 Daltons. The mean MW of chitosan (87% of deacetylation) per an amino group was calculated as 227.9 Daltons.

The mean MW of PEG-chitosan (15% of PEG 2100 w/w., 87% deacetylation) per an amino group was calculated as being 273.9 Daltons.

When the ratio of opposite charge units (or mole ratio) is 1:1, the ratio of DNA to chitosan by weight is 1:0.74 and DNA to PEG-chitosan by weight is 1:0.89. These theoretical values are very close to the experimental results obtained from the macrocalorimetry study. These results indicate that the PEG-chitosan can interact with DNA and form a complex.

The invention provides for the use of a PEG-chitosan material in drug delivery. The PEG moiety may confer advantageous properties, particularly in relation to the physicochemical properties of the chitosan material and the interaction of chitosan with surfaces, particles and DNA where it will be possible to provide a combination of positive charge (from those NH₂ groups of the chitosan that have not been PEGylated) and the PEG-modified amino groups. PEG-chitosan may also improve the paracellular transport of drugs as already described for chitosan itself. Moreover, the PEG-chitosan conjugate may be used to form controlled release materials in the form of microspheres, microparticles and matrices for administration to the gastrointestinal tract, to the vaginal cavity, to the nose and to the buccal cavity.

The conjugate could also be used for the administration of drugs to the eye wherein the chitosan would bind preferably to the mucus. Such an agent may also be used for the treatment of dry eye syndrome, where the PEG moiety provides a steric stabilisation and lubricating effect. These products could be delivered to the eye using eye-drop systems known in the art.

PEG-chitosan complexes with carbohydrates such as alginates, xanthans, dextran sulphate and gellan could be beneficial as gels for drug delivery applications. PEG-chitosan can also be interacted with heparin and other negatively charge macromolecular drugs to form complexes. Nasal and vaginal products as liquids or powders based on PEG-chitosan can be delivered by conventional devices such as spray pumps, powder insufflators or syringes.

The invention provides a pharmaceutical composition comprising a conjugate as described above.

A composition comprising a conjugate as defined above may be used in medicine.

A conjugate of the invention may be used in the manufacture of a medicament for use in medicine.

The invention provides a composition comprising a complex formed between a PEG-chitosan conjugate and a therapeutic agent selected from the group consisting of oligonucleotides and nucleic acids. A composition comprising a complex formed between chitosan and a therapeutic agent selected from the group consisting of oligonucleotides and nucleic acids in which the chitosan is conjugated to a polyethylene glycol moiety is also provided. The therapeutic agent may be intended for use as a drug substance. Alternatively, the therapeutic agent may be intended for use as a prophylatic or therapeutic vaccine. A composition comprising a complex formed between chitosan and DNA in which the chitosan is conjungated to a polyethylene glycol moiety is also provided. Preferably, the PEG-chitosan conjugate is a conjugate as defined above.

The present invention provides use of a PEG-chitosan conjugate to modify the surface of a particulate carrier system intended for medical applications.

The present invention provides use of a PEG-chitosan conjugate to modify the surface of a liposome, emulsion, microcapsule, microsphere or nanoparticle, or to modify the surface of a drug particle.

The present invention provides a particulate carrier system for medical applications the surface of which particles has been modified by a PEG-chitosan conjugate. The present invention also provides a liposome, emulsion, microcapsule, microsphere, nanoparticle or drug particle the surface of which has been modified by a PEG-chitosan conjugate. Such systems, liposomes, emulsions, microcapsules, microspheres, nanoparticles and drug particles may be used for the delivery of drugs antigens, allergens, oligonucleotides, nucleic acids or diagnostic agents.

The present invention provides use of PEG-chitosan conjugate to enhance the solubility of a pharmaceutical agent in water or biological fluid.

The present invention provides use of a PEG-chitosan conjugate for the local treatment of mucosal and topical surfaces of the body of a mammal.

The present invention provides use of a PEG-chitosan conjugate as antibacterial, antifungal or antiviral agent.

Microspheres and microcapsules comprising PEG-chitosan and an additional therapeutic or diagnostic agent are provided.

Controlled release formulations of drugs prepared from the admixture of PEG-chitosan with therapeutic agents are provided. These formulations may be prepared by compression and may be intended for delivery to the gastrointestinal tract. Alternatively, they may be for administration to the nasal, buccal and vaginal cavities.

The present invention also provides the use of the compositions based on PEG-chitosan for the administration of drugs to the eye.

## Claims

1. A composition for the systemic uptake of a drug across a mucosal surface, which composition comprises a PEG-chitosan conjugate.

2. A composition according to Claim 1, wherein the conjugate comprises a chitosan moiety or a derivative thereof and a polyethylene glycol moiety or a derivative thereof which are bonded together via the amino function on the chitosan by the use of an activated chitosan species.

3. A composition according to Claim 2, wherein the activated chitosan species is prepared using bis(acrylamide).

4. A composition according to any one of the preceding claims, in which the chitosan portion of the conjugate has a molecular weight in the range of from 10 kilodalton to 1000 kilodalton.

5. A composition according to any one of the preceding claims, wherein the PEG-chitosan conjugate has the formula:
-[Cm-NR¹ ₂]ₚ- I
wherein:
Cm is the remainder of the monomeric unit making up the chitosan moiety or a derivative of such a monomeric unit;
p is an integer and represents the number of monomeric units of formula -Cm-NR¹₂- in the chitosan moiety,
each R¹ is independently H or a group -A-PEG providing that at least a proportion of the R¹ groups are -A-PEG;
each PEG is independently a polyethylene glycol moiety or a derivative thereof;
each A is independently a linking group having the formula -CH₂CH₂-C(O)-R²-X- which is bonded to the amino nitrogen on the chitosan moiety via the -CH₂CH₂- group and to the PEG moiety via the X group;
R² is a bond or a divalent organic group;
X is oxygen, sulphur or -NR³-; and
R³ is H or an organic group,
or a physiologically acceptable derivative thereof.

6. A composition according to Claim 5, wherein Cm is the remainder of the monomeric unit making up chitosan.

7. A composition according to Claim 5 or Claim 6, wherein p is an integer in the range of from 50 to 6000.

8. A composition according to Claim 7, wherein p is an integer in the range of from 150 to 2000.

9. A composition according to any one of Claims 5 to 8, wherein PEG is a modified polyethylene glycol moiety having the formula R⁴-(OCH₂CH₂)n- wherein R⁴ is an aliphatic, alicyclic or aromatic hydrocarbon group and n is an integer.

10. A composition according to Claim 9, wherein R⁴ is alkyl.

11. A composition according to Claim 10, wherein R⁴ is methyl.

12. A composition according to any one of Claims 5 to 11, wherein R² is a bond or a divalent organic group having the formula: 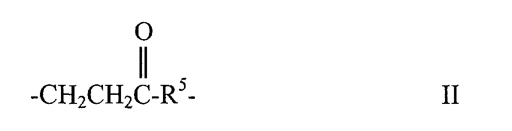 where R⁵ is a divalent organic group which is bonded to the carbonyl (C(O)) group linking group A.

13. A composition according to Claim 12, wherein R⁵ is bonded to the carbonyl group in Formula II and the carbonyl group of linking group A via nitrogen atoms.

14. A composition according to Claim 13, wherein R⁵ is a group having the formula: 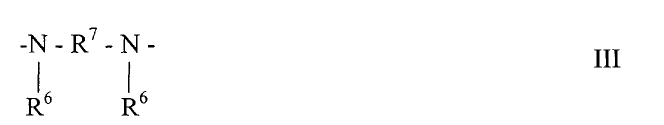 or 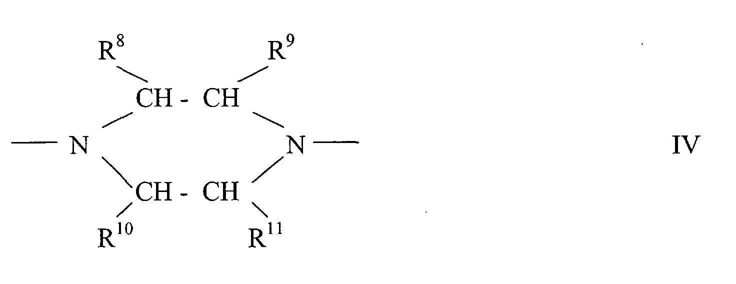 where each R⁶ is independently a linear or branched C₁₋₄ alkyl chain, R⁷ is a divalent organic group and R⁸, R⁹, R¹⁰ and R¹¹ are each independently H or a linear or branched C₁₋₃ alkyl chain.

15. A composition according to Claim 14, wherein R⁷ is a linear or branched C₁₋₄ alkylene chain.

16. A composition according to any one of Claims 5 to 15, wherein X is oxygen or -NR³-.

17. A composition according to any one of Claims 5 to 16, wherein R³ is alkyl or H.

18. A composition according to any one of the preceding claims, wherein the chitosan portion of the conjugate has a molecular weight between 100 kilodalton and 300 kilodalton.

19. A composition according to any one of the preceding claims, wherein the polyethylene glycol content of the conjugate is from 1 to 50% on a weight basis.

20. A composition according to Claim 19, wherein the polyethylene glycol content is from 5 to 20% on a weight basis.

21. A composition according to any one of the preceding claims, wherein the chitosan portion of the conjugate has a degree of deacetylation in the range of from 10 to 99%.

22. A composition according to Claim 21, wherein the chitosan portion has a degree of deacetylation in the range of from 40 to 85%.

23. A composition according to any one of the preceding claims, wherein the polyethylene glycol portion has a molecular weight of between 1 kilodalton and 30 kilodalton.

24. A composition according to Claim 23, wherein the polyethylene glycol portion has a molecular weight between 2 kilodalton and 10 kilodalton.

25. The use of a PEG-chitosan conjugate to enhance the systemic uptake of a drug across a mucosal surface.

26. Use according to Claim 25, wherein the conjugate is as defined in any one of Claims 2 to 24.
